# EUROPEAN PATENT APPLICATION

(11) **EP 4 273 782 A2**
(43) Date of publication of application: **08.11.2023**
(21) Application number: 23176938.1
(22) Date of filing: 22.07.2016
(51) Int. Cl.: G06Q 50/00

(54) **A SYSTEM, DEVICE AND A METHOD FOR PROVIDING A THERAPY OR A CURE FOR CANCER AND OTHER PATHOLOGICAL STATES**

(30) Priority: 25.07.2015 US 201562196939 P; 29.07.2015 US 201562198587 P
(62) Divisional of application: 16742294.8
(71) Applicant: Frost, Habib, 2300 København S (DK)
(72) Inventor: Frost, Habib, 2300 København S (DK)
(74) Representative: Inspicos P/S

(57) **Abstract**

Provided is a method for treatment of pathologies caused by cells that have DNA sequences that differ from DNA of healthy human cells. The method utilises targeting of the sequence differences and effects cleavage of the DNA which does not occur in the healthy genome, whereby the cells containing the targeted DNA are killed or inactivated. Preferred systems for effecting such DNA damage is CRISPR systems where a CAS cleaves DNA adjacent to a sequence recognized by the CRISPR. Also disclosed are methods and a computer system for designing and/or producing the targeting nucleic acids.

## Description

### TECHNICAL FIELD

The invention relates to systems, devices and methods that enables therapeutic intervention against a range of diseases, where the therapy involves killing of certain specifically defined cells. The present method i.e. provides therapy of cancers, viral diseases as well as diseases caused by pathogenic organisms.

### BACKGROUND

Millions of people die of cancer annually. The current therapies of e.g. surgery, chemotherapy, and radiotherapy are harsh and unspecific and kill many healthy cells as well as the cancerous cells that are intended to be eliminated.

Personalized cancer therapies have been attempted, yet few of these have proven to be effective against "any" cancer as each cancer presents itself uniquely for the individual, when taking the fact into account that cancer has a genetic etiology. A cancer in one patient's organ can behave - roughly stated - as an entirely different sub-set of disease in the same place in another patient.

Furthermore, a number of viral infections as well as infections with live pathogens do not have a satisfactory therapy today due to drug resistance or due to unavailability of effective antibiotics that are not harmful to humans at the same time.

There is hence a need for a treatment of cancer that is customized to the specific cancer as it relates to the individual, and there is also a need for therapies that can specifically attack cells infected with virus or cells or pathogenic organisms that are difficult to target with existing antibiotic drugs.

Several studies suggest gene editing in the therapy of cancer, e.g. in the discovery of cancer drug targets by CRISPR-Cas9 screening of protein domains (Shi et al, Nature Biotechnology 2015), by creating new cancer animal models (Mou et al. Genome Medicine 2015), by converting well-known genes related to cancer and converting them into healthy variants using viral vectors, and by using CRISPR-Cas9 in the engineering of CAR-T immunotherapy (chimeric antigen receptor T-cell immunotherapy).

In the known art, gene therapy for a treatment of a cancer is used for *in situ* gene therapy protocols, in which viral vectors are used to transduce specific genes that generate increase in the systemic immunity to the cancer, e.g. in immune-modulatory *in situ* gene therapy of prostate cancer (The et al., Clin Med Res. 2006 Sep; 4(3): 218-227). CAR-T cell immunotherapy is wrought with risks associated with immunotherapy and still poses risks and uncertainty for the patient, e.g. autoimmune disorders, side effects of immunomodulation and death.

### SUMMARY OF THE INVENTION

The present invention is based on the inventor's initial realization that state-of-the-art fast and reliable genome sequences and consequently identification of nucleic acid sequences that are unique to cells (such as cancer cells, virally infected cells, and cells of pathogenic organisms) when compared to healthy cells in an individual can be utilized to design expression vectors that encode recognition and effector molecules to enable selective destruction or neutralization of said cells.

According to findings from the Cancer Genome Project, most cancer cells possess 60 or more mutations. The challenge has been to identify which of these mutations are responsible for particular kinds of cancer as the mutations vary interpersonally - but a vast majority of such mutations are nevertheless unique for the cancer cells and can hence distinguish these cells when targeting them for specific therapy. As for bacteria and virus, the problem of identifying unique sequences for targeting is less of a problem, but it is of course of high relevance to ensure that targeting of a specific viral or bacterial sequence will not lead to undesired off-target effects.

If a recognition molecule with very high reliability/fidelity is able to distinguish a nucleic acid sequence from a cancer cell in an individual from any nucleic acid sequence of a normal cell in the same individual, and if such specific recognition can be utilized to trigger the destruction or neutralization of the "cancer nucleic acid", then the steps of providing an effector cancer treatment in an individual can be broken down into the following simple steps:
1) identification and verification of nucleic acid sequence(s) that appear(s) in the cancer cells but not in healthy cells or at least only in an insignificant population of healthy cells - such an identified sequence is herein termed a "cancer NA sequence";
2) construction of at least one expression vector, which encodes a recognition sequence that is complementary to the cancer NA sequence and also encodes at least one effector molecule, which is capable of destroying or neutralizing nucleic acids that comprise the cancer NA sequence;
3) administration of the expression vector(s) to the patient harboring the cancer so as to effect targeted destruction or neutralization of nucleic acids comprising the cancer NA sequence. In turn, this will assist in destruction or neutralization of the cancer cells.

Since the high number of mutations present in cancer cells vis-à-vis normal cells provide for multiple targets for the recognition sequences, the effect attained when destructing or neutralizing the nucleic acids is equivalent to the multiple DNA disruptions that occur when treating cancer cells with radiation therapy - however, where radiation therapy also causes destruction of DNA in normal cells (and thereby of normal cells), the approach of targeting nucleic acid sequences that are specific for cancer will avoid off-target effects.

The inventor has also realized that the exact same principle can be used in treatment of any type of disease where a pathogen - be it a virally infected cell, a bacterium, or a parasite - is the causative agent for the disease and comprises nucleic acid sequence(s) that are distinct from any nucleic acid sequence in the infected individual. Such a distinct sequence is termed a pathogen NA sequence herein. Construction and administration to the individual of appropriate expression vectors that encode a recognition sequence complementary to pathogen NA sequence and encode an effector molecule capable of destroying or neutralizing nucleic acids that comprise the cancer NA sequence will be able to eradicate cells that comprise the target nucleic acids.

The invention thus provides personalized genetic engineering based treatments that are able to treat a cancer as well as genetic engineering treatments that target virally infected cells or pathogenic infectious agents.

Broadly formulated, the invention provides a system configured to
- interact with patient material, or data from said material, in a predefined reaction pattern
- to provide a design for or synthesize therapeutic genetically engineered biological material
- to follow a predefined reaction pattern based on computer means configured to
- receive input of biological states of sample(s), or data from sample(s), taken from both disease-related material and healthy material sampled from the same patient, hereunder genome, transcriptome, proteome, gene expression, metabolome, and epigenome data, and to
- generate data for design(s) and/or sequence(s) for biological material and/or synthesize biological material based on a predefined pattern that, based on the patient sampled data, is configured to be harmful to the biological states of the disease-related material, and not harmful to the healthy material.

In a first aspect, the present invention relates to a method for treatment of a disease in an animal, such as a human being, the method comprising induction of preferential killing of cells that comprise at least one DNA sequence, which is not present in or is not present in significant amounts in healthy cells of said animal, by administering
1) an effective amount of at least one expression vector, wherein each of said at least one expression vector(s) comprise a first nucleic acid sequence encoding a recognition molecule that specifically recognizes at least one of said at least one DNA sequence(s), and which comprise(s) a second nucleic acid sequence encoding a molecule that selectively disrupts DNA comprising said at least one DNA sequence when it is bound to said recognition molecule, or
2) an effective amount of a at least one first expression vector, wherein each of said at least one expression vector(s) comprise(s) a first nucleic acid sequence encoding a recognition molecule that specifically recognizes at least one of said at least one DNA sequence(s), and an effective amount of at least one second expression vector, which comprises a second nucleic acid sequence encoding a molecule that selectively disrupts DNA comprising said at least one DNA sequence when being bound to said recognition molecule, or
3) an effective amount of at least one recognition molecule that specifically recognizes at least one of said at least one DNA sequence(s) and an effective amount of at least one molecule that selectively disrupts DNA comprising said at least one DNA sequence when being bound to said recognition molecule, or
4) an effective amount of at least one expression vector, which comprises a nucleic acid sequence encoding a recognition molecule that specifically recognizes at least one of said at least one DNA sequence(s) and an effective amount of at least one second molecule that selectively disrupts DNA comprising said at least one DNA sequence when being bound to said recognition molecule, or
5) an effective amount of a at least one recognition molecule that specifically recognizes at least one of said at least one DNA sequence(s) and an effective amount of at least one expression vector, which comprises a nucleic acid sequence encoding a molecule that selectively disrupts DNA comprising said at least one DNA sequence when being bound to said recognition molecule,
wherein administration of said vector(s) and/or molecule(s) defined in any one of 1-5 results in disruption of nucleic acid molecules that comprise said at least one DNA sequence to such a degree that said cells are killed or neutralized.

In a second aspect, the invention relates to a method for designing and optionally preparing a therapeutic means for treatment of a disease in an animal such as a human being, comprising
a) sequencing DNA from a cell or group of cells, where said cell or group of cells is/are from malignant tissue, virus-infected cells, and cells of a pathogenic organism,
b) subsequently comparing DNA sequence information obtained in step a with DNA sequence information from healthy cells of said animal or from a fully sequenced healthy genome of said animal's species,
c) identifying DNA sequences from the cell or group of cells, where said DNA sequences do not appear in the healthy cells of said animal, and
d) designing and optionally preparing a therapeutic means, which at least comprises one of the following
   1) an expression vector, which comprises a first nucleic acid sequence encoding a recognition molecule that specifically recognizes at least one DNA sequence identified in step c, and which comprises a second nucleic acid sequence encoding a molecule that selectively disrupts said at least one DNA sequence identified in step c when it is bound to said recognition molecule, or
   2) a first expression vector, which comprises a first nucleic acid sequence encoding a molecule that specifically recognizes at said least one DNA sequence identified in step c and a second expression vector, which comprises a second nucleic acid sequence encoding a molecule that selectively disrupts said at least one DNA sequence identified in step c when it is bound to said recognition molecule, or
   3) a recognition molecule that specifically recognizes at least one DNA sequence identified in step c, and an expression vector, which comprises a nucleic acid sequence encoding a molecule that selectively disrupts said at least one DNA sequence identified in step c when it is bound to said recognition molecule, or
   4) an expression vector, which comprises a nucleic acid sequence encoding a recognition molecule that specifically recognizes at least one DNA sequence identified in step c, and a molecule that selectively disrupts said at least one DNA sequence identified in step c when being bound to said recognition molecule, or
   5) a recognition molecule that specifically recognizes at least one DNA sequence identified in step c and a molecule that selectively disrupts said at least one DNA sequence identified in step c when it is bound to said recognition molecule.

In a third aspect the invention relates to a computer or computer system for designing and optionally preparing a therapeutic means as described herein, comprising
i) a first computer memory or memory segment comprising DNA sequence data from healthy cells of an animal or from a fully sequenced healthy genome of said animal's species,
ii) a second computer memory or memory segment comprising DNA sequence data from pathology related cells,
iii) executable code adapted to compare DNA sequence data from the second computer memory with the entire set of DNA sequence data in the first memory and identifying DNA sequences from the second computer memory that do not appear in DNA sequence data in the first computer memory,
iv) a third computer memory or memory segment which is adapted to comprise DNA sequence data, or pointers to DNA sequence data, identified by the computer defined in iii, and
v) executable code adapted to design expression vectors based on the 1) DNA sequence data stored or pointed to in the third computer memory or memory segment, and 2) sequence data for nucleic acids encoding effector molecules, and optionally
vi) a nucleic acid synthesizer adapted to synthesize DNA sequences designed by the executable code in v.

### LEGENDS TO THE FIGURE

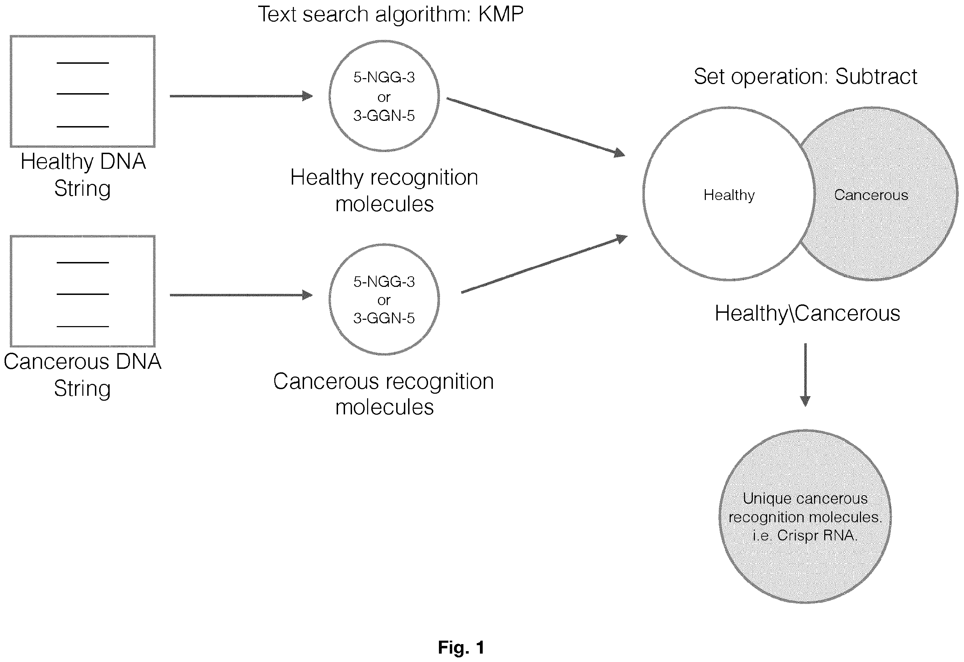

Fig. 1 is a flow-chart illustrating the search and design algorithm disclosed in detail in Example 1.

### DETAILED DISCLOSURE

### Recognition molecules and effector molecules

In much of the discussion and disclosure of the present invention, focus is put on identification of distinguishing nucleic acids derived from cancer/malignant cells. However, as will be understood the present invention is primarily based on the finding that current genome editing technologies such as the CRISPR-Cas-9 system provides for the possibility of specifically targeting and editing any nucleic acid sequence of choice. See for instance WO 2015/089465, which relates to treatment of HBV infection by editing the genome of HBV infected cells and Hsin-Kai Liao et al, Nature Communications 6, Article no. 6413, which relates to a gene edited defense against HIV infection.

As described in the Example, it is possible to search the genome of malignant cells for sequences that can be targeted by a recognition molecule while ensuring that no healthy (normal) cells are targeted. In fact, it is preferred that the method of the first aspect of the invention results in killing and neutralization of malignant cells in a cancer patient, and it is also preferred that the recognition molecule used in the method does not recognized the animal's autologous DNA in healthy (normal) cells.

CRISPR systems have an unsurpassed ease in directing the CRISPR-associated (Cas) proteins (such as Cas9 and any of the other effector molecules discussed in the context of CRISPR herein) to multiple gene targets by providing guide RNA sequences complementary to the target sites. Target sites for CRISPR/Cas9 systems can be found near most genomic loci; the only requirement is that the target sequence, matching the guide strand RNA, is followed by a protospacer adjacent motif (PAM) sequence. For *Streptococcus pyogenes* (Sp) Cas9, this is any nucleotide followed by a pair of guanines (NGG). However, Francisella novicida Cas9 has been engineered to recognize the PAM YG (any pyrimidine followed by guanine), and Cpf1 of Francisella novicida recognizes the PAM TTN or YTN.

Hence, is preferred that the recognition molecule is a nucleic acid, which recognizes said at least one DNA sequence, which is targeted, via base-pairing. Typically, the recognition molecule is a crRNA and the molecule that selectively disrupts said at least one DNA sequence when being bound to said recognition molecule is a CRISPR-associated protein (Cas), i.e. a nuclease. This Cas can be any suitable Cas, but may e.g. be Cas9, eSpCas9, SpCas9-HF, and Cpf1.

The selection of the target sites and choice of CRISPR System for the therapy may be further optimized by prioritizing the targets via a CRISPR system target site and off-target site(s) predictive activity scorer, such as e.g. the SgRNA Scorer 1.0 made available by Harvard University.

While it is preferred according to the present invention that the cells that are targeted by a recognition sequence are lethally damaged due to the disruption of multiple DNA molecules, the therapy of the invention will be effective even in the event that the targeted cells are merely "neutralized".

When utilizing CRISPR systems for specific recognition of diverging DNA sequences in malignant cells, the fidelity of the recognition can be increased by employing recognition sequences that include modification in the backbone of the recognition sequence. For instance, stability of the duplex between a recognition sequence and its target DNA may be improved by using recognition sequences that include modified nucleotides. An example is locked nucleic acids (LNA); oligonucleotides modified with LNA (a ribose modification) can form stable duplexes between relatively short complimentary nucleic sequences and in this manner it can be ensured that the risk of off-target effects is minimized. Other possibilities are the use of phosphate backbone variants, such as phosphodiester and phosphorothioate internucleotide linkage modifications, ribose variants, such as LNA, 2'OMe, 2'-fluoro RNA (2'F) and 2'MOE; and non-ribose backbones variants such as PMO and PNA.

Such approaches will require that that the recognition sequence is provided by means of direct administration because the modified nucleic acids are not expression products.

In preferred embodiments of the invention the design(s) and/or sequence(s) of CRISPR system(s) are including, but not limited to, CRISPR-Cas9 system(s), CRISPR-eSpCas9 system(s), and SpCas9-HF system(s) and CRISPR-Cpf1 system(s), alternatively homologous recombination, RNA interference (RNAi), zinc-finger nucleases (ZFNs), transcription-activator like effector nucleases (TALENs) and other future ways to make precise, targeted changes to the genome. This means that the effector molecule (nuclease) for instance can be selected between Cas9, eSpCas9, SpCas9-HF, and Cpf1. Since a number of these exert minimal off-target effects, the safety profile of the present invention predominantly relies on correct identification of unique DNA sequences not found in the normal cells.

An attractive feature of the present invention is that it may be tested *in vitro* prior to subjecting the patient to the treatment thereby further increasing safety. When a selection of recognition sequences have been designed, they can be administered to isolated cancer cells from the patient and to isolated normal cells from the patient - only in the event that the DNA disrupting effect is substantially confined to the cancer cells should the therapy be invoked in the patient. This approach is in particular relevant in those embodiments disclosed herein where the DNA of the cancer cells are compared to a standard healthy genome - as a safety measure it is by this approach ensured that the patients normal cells will not by chance include one or more of the DNA targets that are believed to be specific for the cancer.

So, in an embodiment of the invention involves that prior to *in vivo* administration of the synthesized personalized therapy, the therapy is tested, optimized and validated *in vitro* on healthy cells and diseased cells. The expected outcome is measured by a nucleotide-resolution DNA double-strand breaks mapping, such as e.g. the direct *in situ* breaks labeling, enrichment on streptavidin, and next-generation sequencing (BLESS). Alternatively, simple cell-survival may be determined *in vitro.*

Bioinformatics search algorithms exist in the known art that when supplied with a gene or whole genome can find all the possible 20-base segments located near a PAM, rank them based on their uniqueness in the genome and other parameters, and generates a list of guide RNAs that gets you there, such as e.g. Harvard University's CHOPCHOP or Yale University's CRISPRscan. In the present context, the discussion of DNA derived from malignant cells in a patient can hence equally well be applied to DNA derived from any pathogen or cell involved in a pathology, as long as said DNA is not identical to DNA found in the healthy cells of the individual to be treated. In essence, only the choice of recognition molecule (e.g. its nucleic acid sequence) needs to be changed when desiring to target a particular DNA of a cancer or other pathology-related cell - in addition, the choice of means for ensuring entry of the recognition molecule into the target cell will normally have to be optimized for the particular target cell. To conclude on this, all disclosure in the present application that relates to the design of the recognition molecule in the context of cancer may be employed in an analogous manner for other pathologies.

In one embodiment of the invention, the provided design(s) and/or sequence(s) is used in the synthesis of CRISPR system(s), alternatively homologous recombination, RNA interference (RNAi), zinc-finger nucleases (ZFNs), transcription-activator like effector nucleases (TALENs) and other future ways to make precise, targeted changes to the genome. The currently preferred system is a CRISPR-Cas9 system, which has today been developed into a versatile system for gene editing of eukaryotic (including human) genomes.

If using a CRISPR-Cas9 system, the only other prerequisite is that the sequence that is to be cleaved according to the invention is followed downstream, or upstream by a proto-spacer adjacent motif (PAM) sequence (such as 5'-NGG-3').

As will be clear from the claims, the treatment of the invention can take many practical forms and merely has to ensure that the target cell at the same time 1) harbors a recognition molecule (such as at least one CRISPR having a DNA sequence which is complementary to a cancer NA sequence or a pathogen NA sequence) and an effector molecule (such as a Cas9). Both molecules can be encoded by one and the same expression vector, the may be encoded by separate expression vectors, or only one is encoded by an expression vector whereas the other molecule is administered directly, or they may be both administered directly.

Preferred expression vectors are adenovirus, adeno-associated virus, or lentivirus but any vector format that can provide cellular presence of the recognition and effector molecules can be utilized. Hence, methods generally applied in gene therapy to effect transfection into target cells are practical according to the present invention. For instance, if using longer, optionally modified, nucleic acids that are not introduced in a viral vector but instead as plasmids, it is according to the invention attractive to formulate these so that they will be taken up by target cells - liposome formulations constitute one possibility, but the nucleic acids may also be modified directly by e.g. linking to lipids such as cholesterol.

As will be understood, the preferred CRISPR-Cas9 systems are normally used as gene editing tools rather than merely introducing breaks in target DNA. However, both the gene editing as well as the gene break approaches are useful when killing or neutralizing cells according to the present invention.

By "neutralization" is meant that a cell is inactivated due to damage of its DNA, at least to the extent that it cannot proliferate (in cancers this will mean that the cancer's growth is interrupted) and/or that it cannot replicate its genome. Other possibilities are that the cells become more immunogenic due to the appearance of immunogenic expression products and therefore become inactive/killed as a consequence of an immune reaction. It is also possible that the DNA damage induces at least partial reversion to a benign phenotype, meaning that the cells loose their ability to metastasize or their ability to invade tissue. Finally, the cells may also become generally more sensitive to other anticancer therapies with which the principles of the present invention can be combined. It is, however, preferred that targeted cells are killed as a consequence of the DNA disruption induced.

In one of the simplest embodiments, the method of therapy of the invention utilizes one or more recognition molecules (such as CRISPRs) that have been designed to target as many important chromosomes in the target cell as possible. Introduction of a sufficient number of breaks in the chromosomal DNA will have the effect that the cell subsequently is not viable; as detailed above, the effect on the targeted cells is equivalent to the effect of radiation therapy.

Also, frameshift mutations may be introduced site-specifically by the method of the invention, e.g. using a properly designed CRISPR-Cas9 or similar system. A frameshift is a genetic mutation caused by indels (insertions or deletions) of a number of nucleotides in a DNA sequence that is not divisible by three. Due to the triplet nature of gene expression by codons, the insertion or deletion can change the reading frame (the grouping of the codons), resulting in a completely different protein expression product compared to that of the non-mutated gene. The earlier in the sequence the deletion or insertion occurs, the more altered the protein will be.

If an indel frameshift occurs in one or more vital proteins of the target cell, and the repair mechanism(s) are inhibited, as is the case in cancer, the frameshift is incompatible with continued life for the cell.

A frameshift mutation is not the same as a single-nucleotide polymorphism in which a nucleotide is replaced, rather than inserted or deleted.

This approach is another way in which an organism could be disrupted.

If several sites are targeted at the same time, e.g. for cutting out a larger piece of the genome organism, as is the case when doing recombinant DNA engineering, a larger impact through above-mentioned damage could be induced.

Another way a cell may be killed is by introducing one or several mutations that introduce stops stop codons ("UAA", "UGA" or "UAG") into the sequence. The polypeptide being created could be abnormally short or abnormally long, and will most likely not be functional.

In interesting embodiments Crispr-Cas9 systems or similar systems are used, which insert a death gene or killing mechanism into the target cells, so as to effectively kill the cancer the fastest.

A more simple approach relies on the introduction of one or multiple disruptions of the genome of the target cells, so as to generally interfere with the metabolism of the cell.

In another embodiment of the invention, the Crispr-Cas9 or similar systems used deletes one or multiple base pairs of the target cell genome, so as to disturb the metabolism of the cell.

In another embodiment of the invention the Crispr-Cas9 or similar systems modify or insert one or multiple genes into the target cell genome, so as to be able to visually or electromagnetically detect the cancer cells from the perspective of a surgical procedure, e.g. through fluorescent dye.

In another embodiment, the Crispr-Cas9 system(s) is configured to produce a single-nucleotide polymorphism in which a nucleotide is replaced, rather than inserted or deleted.

In another embodiment, several sites are targeted at the same time, e.g. for cutting out a larger piece of the genome organism, as is the case when doing recombinant DNA engineering that is deleterious, a larger impact through above-mentioned damage could be induced.

In another embodiment of the invention, the crispr-Cas9 system(s) is configured to introduce one or several mutations that introduce stops stop codon ("UAA", "UGA" or "UAG") into the sequence of the cancerous cells.

As will be apparent from the claims, the method of therapy of the invention finds broad use as long as genetic material which is not present in the treated individual's own healthy cells is indeed present in pathology-related cells. Apart from cancers, the present invention allows for targeting of "difficult" bacterial and other infections such as e.g. tuberculosis or specific targeting of bacteria or other pathogenic organisms that have either developed drug resistance or are notoriously difficult to control/eradicate. Certain infections do not have a current effective cure: malaria and a number of other parasitic diseases (schistosomiasis and infections with Echinococcus multilocularis are examples), and also a number of viral diseases.

With respect to viral diseases, it will be possibly to employ the present invention to target only those cells in an individual, which comprise DNA derived from the virus (in those cases where the virus utilizes the biochemical machinery of an infected cell to produce viral proteins from DNA). Since the normal immunological defense against such viral infections is induction of killing of infected cells, the presently disclosed approach is simply one that attains the same end result in a different way. Interesting targets would be HIV infection, as well as HBV and HCV.

When it comes to other infections, the identification of target DNA is more or less trivial due to the large differences between human DNA sequences and the sequences found in bacteria, fungi and parasites. In those cases, the main task is to select an optimized means for effecting introduction of the recognition and effector molecules into the target cells - in the case of bacteria, phage may be used as a vector, whereas other means, e.g. a mycovirus or a nematode virus, may be useful to introduce expression vectors into fungi and parasites.

A separate embodiment of the first aspect of the invention integrates the active principle(s) into a preventive or therapeutic depot, i.e. "a vaccine", in the form of an injection, patch, pellet or implant that disposes a continued dose of the active principle(s) into a tissue, fluid compartment or blood vessel of the animal treated, either preventively before a disease has been acquired or after diagnosis. This embodiment entails use of state-of-the-art technology for depositing a drug and ensure sustained release.

### Method and means for identifying and preparing therapeutic means

In preferred embodiments the method of the second aspect as well as the computer means of the invention both cancerous or pathogen genomes are compared with healthy genomes to find areas in the sequences that differ in such a way that the differences can direct the design of the RNA part of a Crispr-Cas9, so that it selectively attacks the cancer or pathology related cells and not the healthy cells. This designing of the sequence of said RNA can be achieved by aggregating one or multiple samples from one or multiple healthy cells and from one or multiple cancer cells, to ensure that the genome is highly representative of the healthy genome, and to ensure that the difference(s) is highly representative of the genome of the cancer. Potentially also that the difference(s) are present in multiple generations of the cancer, so as to appear in a majority of the cancer cells. This is due to rapid mutations potentially occurring.

The method of the second aspect and the computer means is thus set up to use the provided design(s) and/or sequence(s) in the synthesis of CRISPR-Cas9 system(s) (such as those detailed herein), alternatively homologous recombination, RNA interference (RNAi), zinc-finger nucleases (ZFNs), and transcription-activator like effector nucleases (TALENs) to make precise, targeted changes to the genome.

In general, in the method of the invention where useful sequences to target are identified, it is relevant to have access to the entire sequence information for normal cells in the individual to be treated. However, this may be dispensed with; instead an already sequenced (healthy) genome may be used even though this is slightly less safe due to the risk that the individual to be treated may comprise sequences in common with the disease sequences, whereas this is not the case for the already sequenced genome. However, as indicated herein, it is possible to perform an initial safety evaluation *in vitro* by subjecting malignant cells and healthy cells from the individual to be treated to the combination of the recognition and the molecule that disrupts the targeted DNA sequence when it is bound to the recognition molecule.

When sequencing of the disease-related cell DNA starts, it may take more or less time before the entire genome is sequenced, but during the process useful target sequences may be continuously identified - perhaps even at a very early time. Each time a sequence is identified, it is added to a list of potential target sequences which may be ranked and re-ranked with reference to already included sequences and sequences that are continuously included - the criteria used to rank the sequences include an evaluation of presence of PAMs, presence of the sequence in vital chromosomes, etc., see below.

It may also be convenient to compare the cancer DNA sequences with one or more completely sequenced normal genomes and search for differences vis-à-vis highly conserved genes in the previously sequence genomes.

In addition, a list of approximately 200 "common" gene mutations that are related and specific for a number of cancers are already known. If such a mutation is identified as part of the identification process, such sequences will as a rule be ranked in a top tier for later preparation of the therapeutic means when the target is a cancer - first and foremost because it is already known that these sequences are cancer specific and inclusion of a recognition sequence that targets such a cancer-specific sequence will be a safe choice.

In one embodiment of the invention the output of the system is integrated into an adenovirus (i.e. adeno-associated virus) or lentivirus as the treatment vector.

In one embodiment of the invention, the computer means are configured to search and detect sequences in the cancer genome that differ from the healthy genome with 1-20 nucleotides at the same location in between the genomes, and configured to find such sites that are followed downstream, or upstream, by the proto-spacer adjacent motif (PAM) sequence (5'-NGG-3'). The system is configured to - in case of finding multiple of such sites - to rank the finds based on the ones with the most differences, and alternatively based on a database of knowledge of the importance of the genes in question. The system is configured to at least one of the following: 1) Rank the findings highest with the biggest difference between the healthy and the cancerous/pathogen-related 2) Rank the highest finding(s) whose use is known to have the highest impact based on the predefined database of knowledge. 3) Be able to present the location of these findings. 4) Generate design for and/or synthesize a treatment based on these findings by mirroring the finding into a sequence of RNA, that when synthesized is the recipe for a unit that can be included in a Crispr-Cas9 system, that will specifically be targeting the cancer cells, and will not be targeting the healthy cells. 5) Produce several outputs, such as several Crispr-Cas9 systems, that attack several different points within the targeted cancerous material, e.g. multiple points of the genome of the cancer, with the intent of disrupting a larger part of the cancerous material. In the case of attacking several points at once in a cancer cell, the intention is to cut out a larger piece of the genome to cause more damage.

In one embodiment of the invention, the genetic input is configured to use a Liquid Biopsy technique to extract the genetic material of the cancer from a blood sample of the patient.

In another embodiment of the invention, the computer means takes this database of differences between the genomes, and identifies differences that include a PAM site, are adjacent to or in proximity to a PAM site, e.g. differences between two genomes that are 1-20 nucleotides away from a PAM site. In essence, even a few mismatches between as normal DNA sequence and one found in a cancer may be utilized as target. In particular interesting embodiments where a longer mismatch is identified, multiple, partially overlapping recognition sequences can be designed so as to enable a multitude of possible breaks in a cancer cell's DNA.

In one embodiment, the machine can receive one or more samples of healthy and one or more samples of diseased cells to ensure identification of a site that has high probability of representing a segment of diseased DNA that is representative of different generations of diseased cells.

To improve the existing cancer treatments, and to enable a safe vector with high specificity for the cancer only, the present invention provides a system that can aggregate several data samples regarding the cancerous and non-cancerous material, so as to increase the safety level and specificity regarding the probability of what the data represents for the respective material types.

Due to the configuration and the computer means, the system allows for a highly specific cancer treatment, that take the specifications of the patients healthy cells and the configuration of the patient's cancer cells into account.

In one embodiment of the invention, the computer means is configured to read digital documents containing at least genomic data and generate digital documents containing at least genomic data.

Due to the computer means of the system and the ability to receive and interpret patient data, the device can enable a cancer treatment that takes the genome of the individual's actual cancer specifications into account, in the design of the targeted genetically engineered treatment.

In another embodiment, the computer system is configured in a way to itself generate the information defining the treatment vector and its design based on the individual patient's genome - this merely requires that the computer is pre-programmed with the sequence information for one or more expression vectors into which the sequences encoding recognition molecules and effector molecules can be inserted. Optionally, the computer system can be linked or deliver synthesis input to a nucleic acid synthesizer, which can produce the expression vectors useful in the invention.

The device facilitates a safe procedure in which a computer interacts with not only the patient based data, but also a database of knowledge regarding optimal strategies in which to harm the cancer cells.

In one embodiment, the database includes the information below and information about genetic sites known to be universal in humans, and their genetic products.

As will be apparent from the claims, the presently described computer system and the related method is useful in method(s) for providing a therapy or a cure for cancer, said method(s) comprising to interact with patient material, or data from said material, in a predefined reaction pattern:
- to provide a design for or synthesize therapeutic genetically engineered biological material
- to follow a predefined reaction pattern based on computer means configured to
   - Receive input of biological states of sample(s), or data from sample(s), taken from both non-cancerous material and cancerous material sampled from the same patient, hereunder genome, transcriptome, proteome, gene expression, metabolome and epigenome.
   - Generate data for design(s) and/or sequence(s) for biological material and/or synthesize biological material based on a predefined pattern that, based on the patient sampled data, is configured to be harmful to the biological states of the cancerous material, and not harmful to the non-cancerous material.

These methods may further utilize one or more of the aspects of the system mentioned earlier, as will also be apparent from earlier paragraphs.

Having thus described in detail a preferred embodiment of the of the present invention, it is to be appreciated and will be apparent to those skilled in the art that many changes not exemplified in the detailed description of the invention could be made without altering the inventive concepts and principles embodied therein. It is also to be appreciated that numerous embodiments incorporating only part of the preferred embodiment are possible which do not alter, with respect to those parts, the inventive concepts and principles embodied therein. The presented embodiments are therefore to be considered in all respects exemplary and/or illustrative and not restrictive, the scope of the invention being indicated by the appended claims, and all alternate embodiments and changes to the embodiments shown herein which come within the meaning and range of equivalency of the appended claims are therefore to be embraced therein.

It will hence be understood that the present invention relates in particular to the following numbered embodiments (NE):
NE1. A method for treatment of a disease in an animal, such as a human being, the method comprising induction of preferential killing of cells that comprise at least one DNA sequence, which is not present in or is not present in significant amounts in healthy cells of said animal, by administering
   1) an effective amount of at least one expression vector, wherein each of said at least one expression vector(s) comprise a first nucleic acid sequence encoding a recognition molecule that specifically recognizes at least one of said at least one DNA sequence(s), and which comprise(s) a second nucleic acid sequence encoding a molecule that selectively disrupts DNA comprising said at least one DNA sequence when it is bound to said recognition molecule, or
   2) an effective amount of a at least one first expression vector, wherein each of said at least one expression vector(s) comprise(s) a first nucleic acid sequence encoding a recognition molecule that specifically recognizes at least one of said at least one DNA sequence(s), and an effective amount of at least one second expression vector, which comprises a second nucleic acid sequence encoding a molecule that selectively disrupts DNA comprising said at least one DNA sequence when being bound to said recognition molecule, or
   3) an effective amount of at least one recognition molecule that specifically recognizes at least one of said at least one DNA sequence(s) and an effective amount of at least one molecule that selectively disrupts DNA comprising said at least one DNA sequence when being bound to said recognition molecule, or
   4) an effective amount of at least one expression vector, which comprises a nucleic acid sequence encoding a recognition molecule that specifically recognizes at least one of said at least one DNA sequence(s) and an effective amount of at least one second molecule that selectively disrupts DNA comprising said at least one DNA sequence when being bound to said recognition molecule, or
   5) an effective amount of a at least one recognition molecule that specifically recognizes at least one of said at least one DNA sequence(s) and an effective amount of at least one expression vector, which comprises a nucleic acid sequence encoding a molecule that selectively disrupts DNA comprising said at least one DNA sequence when being bound to said recognition molecule,
   wherein administration of said vector(s) and/or molecule(s) defined in any one of 1-5 results in disruption of nucleic acid molecules that comprise said at least one DNA sequence to such a degree that said cells are killed or neutralized.
NE2. The method of numbered embodiment NE1, where the animal is not exposed to any recognition molecule that recognizes the animal's autologous DNA in healthy cells.
NE3. The method of numbered embodiment NE1 or NE2, wherein the disease is a cancer and wherein the cells killed or neutralized are malignant cells.
NE4. The method of numbered embodiment NE1 or NE2, wherein the disease is an infection selected from infection with bacteria, infection with fungi, and infection with a parasite, and where the cells killed or neutralized are cells from the infectious agent.
NE5. The method according to numbered embodiment NE1 or NE2, wherein the disease is a viral infection wherein cells in the animal comprise DNA derived from the infectious virus and wherein the cells killed or neutralized are those that comprise DNA derived from the infectious virus.
NE6. The method according to any one of the preceding numbered embodiments, wherein the recognition molecule is a nucleic acid, which recognizes said at least one DNA sequence via base-pairing.
NE7. The method according to numbered embodiment NE6, wherein the recognition molecule is a crRNA and wherein said molecule that selectively disrupts said at least one DNA sequence when being bound to said recognition molecule is a CRISPR-associated protein (Cas).
NE8. The method of numbered embodiment NE7, wherein the Cas is one of the following: Cas9, eSpCas9, SpCas9-HF, and Cpf1.
NE9. A method for designing and optionally preparing a therapeutic means for treatment of a disease in an animal such as a human being, comprising
   a) sequencing DNA from a cell or group of cells, where said cell or group of cells is/are from malignant tissue, virus-infected cells, and cells of a pathogenic organism,
   b) subsequently comparing DNA sequence information obtained in step a with DNA sequence information from healthy cells of said animal or from a fully sequenced healthy genome of said animal's species,
   c) identifying DNA sequences from the cell or group of cells, where said DNA sequences do not appear in the healthy cells of said animal, and
   d) designing and optionally preparing a therapeutic means, which at least comprises one of the following
      1) an expression vector, which comprises a first nucleic acid sequence encoding a recognition molecule that specifically recognizes at least one DNA sequence identified in step c, and which comprises a second nucleic acid sequence encoding a molecule that selectively disrupts said at least one DNA sequence identified in step c when it is bound to said recognition molecule, or
      2) a first expression vector, which comprises a first nucleic acid sequence encoding a molecule that specifically recognizes at said least one DNA sequence identified in step c and a second expression vector, which comprises a second nucleic acid sequence encoding a molecule that selectively disrupts said at least one DNA sequence identified in step c when it is bound to said recognition molecule, or
      3) a recognition molecule that specifically recognizes at least one DNA sequence identified in step c, and an expression vector, which comprises a nucleic acid sequence encoding a molecule that selectively disrupts said at least one DNA sequence identified in step c when it is bound to said recognition molecule, or
      4) an expression vector, which comprises a nucleic acid sequence encoding a recognition molecule that specifically recognizes at least one DNA sequence identified in step c, and a molecule that selectively disrupts said at least one DNA sequence identified in step c when being bound to said recognition molecule, or
      5) a recognition molecule that specifically recognizes at least one DNA sequence identified in step c and a molecule that selectively disrupts said at least one DNA sequence identified in step c when it is bound to said recognition molecule.
NE10. The method according to numbered embodiment NE9, wherein step c also includes ranking the sequences identified.
NE11. A computer or computer system for designing and optionally preparing a therapeutic means as defined in numbered embodiment NE9 or NE10, comprising
   i) a first computer memory or memory segment comprising DNA sequence data from healthy cells of an animal or from a fully sequenced healthy genome of said animal's species,
   ii) a second computer memory or memory segment comprising DNA sequence data from pathology related cells,
   iii) executable code adapted to compare DNA sequence data from the second computer memory with the entire set of DNA sequence data in the first memory and identifying DNA sequences from the second computer memory that do not appear in DNA sequence data in the first computer memory,
   iv) a third computer memory or memory segment which is adapted to comprise DNA sequence data, or pointers to DNA sequence data, identified by the computer defined in iii, and
   v) executable code adapted to design expression vectors based on the 1) DNA sequence data stored or pointed to in the third computer memory or memory segment, and 2) sequence data for nucleic acids encoding effector molecules, and optionally
   vi) a nucleic acid synthesizer adapted to synthesize DNA sequences designed by the executable code in v.
NE12. The computer or computer system according to numbered embodiment NE11, configured to use the provided design(s) and/or sequence(s) in the synthesis of CRISPR-Cas9 system(s), such as those involving Cas9, eSpCas9, SpCas9-HF, or Cpf1, alternatively homologous recombination, RNA interference (RNAi), zinc-finger nucleases (ZFNs), and transcription-activator like effector nucleases (TALENs) to make precise, targeted changes to the genome.
NE13. The computer or computer system according to numbered embodiment NE11 or NE12, which carries out the method according to numbered embodiment NE10, steps b-d.
NE14. A composition for use in a method for treatment of a disease in an animal, such as a human being, the method comprising induction of preferential killing of cells that comprise at least one DNA sequence, which is not present in or is not present in significant amounts in healthy cells of said animal, wherein said composition comprises
   1) an effective amount of at least one expression vector, wherein each of said at least one expression vector(s) comprise a first nucleic acid sequence encoding a recognition molecule that specifically recognizes at least one of said at least one DNA sequence(s), and which comprise(s) a second nucleic acid sequence encoding a molecule that selectively disrupts DNA comprising said at least one DNA sequence when it is bound to said recognition molecule, or
   2) an effective amount of a at least one first expression vector, wherein each of said at least one expression vector(s) comprise(s) a first nucleic acid sequence encoding a recognition molecule that specifically recognizes at least one of said at least one DNA sequence(s), and an effective amount of at least one second expression vector, which comprises a second nucleic acid sequence encoding a molecule that selectively disrupts DNA comprising said at least one DNA sequence when being bound to said recognition molecule, or
   3) an effective amount of at least one recognition molecule that specifically recognizes at least one of said at least one DNA sequence(s) and an effective amount of at least one molecule that selectively disrupts DNA comprising said at least one DNA sequence when being bound to said recognition molecule, or
   4) an effective amount of at least one expression vector, which comprises a nucleic acid sequence encoding a recognition molecule that specifically recognizes at least one of said at least one DNA sequence(s) and an effective amount of at least one second molecule that selectively disrupts DNA comprising said at least one DNA sequence when being bound to said recognition molecule, or
   5) an effective amount of a at least one recognition molecule that specifically recognizes at least one of said at least one DNA sequence(s) and an effective amount of at least one expression vector, which comprises a nucleic acid sequence encoding a molecule that selectively disrupts DNA comprising said at least one DNA sequence when being bound to said recognition molecule,
   wherein administration of said vector(s) and/or molecule(s) defined in any one of 1-5 results in disruption of nucleic acid molecules that comprise said at least one DNA sequence to such a degree that said cells are killed or neutralized.
NE15. The composition for use according to numbered embodiment NE14, wherein the method is as defined in any one of numbered embodiments NE2-NE8.

### DETAILED DESCRIPTION OF EMBODIMENTS OF THE INVENTION

In the following, embodiments of the invention will be described in further details with reference to the drawing in which:
Fig. 1 illustrates a stepwise schematic of the computation.

### EXAMPLE 1

### A bioinformatics proof-of-concept experiment

Fig. 1 illustrates a stepwise schematic of the computation provided below in detail.

The input parameters for the computer system are DNA sequences from a group of cells, where said cells represent healthy tissue, and DNA sequences from a group of cells, where said cells represent malignant tissue in this experiment. The following algorithm could be supplied with input parameters from virus-infected cells and cells of a pathogenic organism.

The sequencing data could be whole genome, partial and/or deep DNA or RNA sequencing.

First the PAM sequences of the healthy, or targeted cells, are identified using KMP (Knuth-Morris-Pratt) text search. The PAM sequence in this experiment represented the prospective use of Crispr-Cas9 and was thus 5'-NGG-3'. The PAM sequences, including the following or preceding DNA nucleotides with a length of the desired complementary recognition molecule, e.g. Crispr RNA, are stored in list form for later comparison. A trial run was executed using a custom algorithm made with the BioPython open source project and a chromosome 2 sample from a healthy and cancerous genome from the same individual found in AWS 1000 genomes project's database (refl NW_004929308.1).

Thereafter, the chromosome 2 sample sequences were inserted into their own respective Set (or Bag) data structure. The Set allows for duplicate values, such that more occurring sequences are not ignored, and can take part in a partial match analysis. A Set data structure is used for its constant O(1) runtime, essential for larger DNA sequences.

As the third step, the two respective sets are computed into a third result set with a subtract (or difference) operation. The difference operation, results in a set of complementary recognition molecules, unique to the cancer cell to be targeted, and not existing in the healthy cell. In the conducted experiment out of 230 million nucleotides, including overlapping duplicates, 8 *million* potential targets were identified with the PAM occurring at variable positions within the target sequence.

Further analysis can then be performed on the resulting set of sequences, unique to the cancer cell, or healthy cell, if desired, such as ranking of their uniqueness, ranking of target sequences that correlate with targets within sequences known to correspond with parts of the exome, ranking of the targets based on their predictive activity scorer, such as via the SgRNA Scorer 1.0; the ranking of the targets further optimized based on their usability for e.g. designing LNA, ranking based on their viability to be expressed in an specific vector, e.g. an adeno-associated virus or another vector format that can provide cellular presence of the recognition and effector molecules.

## Claims

1. A composition for use in a method for treatment of cancer in an animal, such as a human being, the method comprising induction of preferential killing of malignant cells that comprise at least one DNA sequence, which is not present in or is not present in significant amounts in healthy cells of said animal, wherein said composition comprises
1) an effective amount of at least one expression vector, wherein each of said at least one expression vector(s) comprise(s) a first nucleic acid sequence encoding a recognition molecule that specifically recognizes at least one of said at least one DNA sequence(s), and which comprise(s) a second nucleic acid sequence encoding a molecule that selectively disrupts DNA comprising said at least one DNA sequence when it is bound to said recognition molecule, or
2) an effective amount of at least one first expression vector, wherein each of said at least one expression vector(s) comprise(s) a first nucleic acid sequence encoding a recognition molecule that specifically recognizes at least one of said at least one DNA sequence(s), and an effective amount of at least one second expression vector, which comprises a second nucleic acid sequence encoding a molecule that selectively disrupts DNA comprising said at least one DNA sequence when being bound to said recognition molecule, or
3) an effective amount of at least one recognition molecule that specifically recognizes at least one of said at least one DNA sequence(s) and an effective amount of at least one molecule that selectively disrupts DNA comprising said at least one DNA sequence when being bound to said recognition molecule, or
4) an effective amount of at least one expression vector, which comprises a nucleic acid sequence encoding a recognition molecule that specifically recognizes at least one of said at least one DNA sequence(s) and an effective amount of at least one second molecule that selectively disrupts DNA comprising said at least one DNA sequence when being bound to said recognition molecule, or
5) an effective amount of at least one recognition molecule that specifically recognizes at least one of said at least one DNA sequence(s) and an effective amount of at least one expression vector, which comprises a nucleic acid sequence encoding a molecule that selectively disrupts DNA comprising said at least one DNA sequence when being bound to said recognition molecule,
wherein administration of said vector(s) and/or molecule(s) defined in any one of 1-5 results in disruption of nucleic acid molecules that comprise said at least one DNA sequence to such a degree that said malignant cells are killed or neutralized, and wherein
said at least on DNA sequence in the malignant cells is followed downstream or upstream by a proto-spacer adjacent motif (PAM) sequence.

2. The composition for the use according to claim 1, where the animal is not exposed to any recognition molecule that recognizes the animal's autologous DNA in healthy cells.

3. The composition for the use according to claim 1 or 2, wherein the recognition molecule is a nucleic acid, which recognizes said at least one DNA sequence via base-pairing.

4. The composition according for the use according to claim 3, wherein the recognition molecule is a crRNA and wherein said molecule that selectively disrupts said at least one DNA sequence when being bound to said recognition molecule is a CRISPR-associated protein (Cas).

5. The composition for the use according to claim 4, wherein the Cas is one of the following: Cas9, eSpCas9, SpCas9-HF, and Cpf1.

6. The composition for the use according to any one of the preceding claims, wherein said at least on DNA sequence in the malignant cells is followed 1-20 nucleotides downstream or upstream by a proto-spacer adjacent motif (PAM) sequence.

7. A method for designing and optionally preparing a therapeutic means for treatment of cancer in an animal such as a human being, comprising
a) sequencing DNA from a cell or group of cells, where said cell or group of cells is/are from malignant tissue,
b) subsequently comparing DNA sequence information obtained in step a with DNA sequence information from healthy cells of said animal or from a fully sequenced healthy genome of said animal's species,
c) identifying DNA sequences from the cell or group of cells, where said DNA sequences do not appear in the healthy cells of said animal and wherein said DNA sequences are followed downstream or upstream by a proto-spacer adjacent motif (PAM) sequence, and
d) designing and optionally preparing a therapeutic means, which at least comprises one of the following
1) an expression vector, which comprises a first nucleic acid sequence encoding a recognition molecule that specifically recognizes at least one DNA sequence identified in step c, and which comprises a second nucleic acid sequence encoding a molecule that selectively disrupts said at least one DNA sequence identified in step c when it is bound to said recognition molecule, or
2) a first expression vector, which comprises a first nucleic acid sequence encoding a molecule that specifically recognizes at said least one DNA sequence identified in step c and a second expression vector, which comprises a second nucleic acid sequence encoding a molecule that selectively disrupts said at least one DNA sequence identified in step c when it is bound to said recognition molecule, or
3) a recognition molecule that specifically recognizes at least one DNA sequence identified in step c, and an expression vector, which comprises a nucleic acid sequence encoding a molecule that selectively disrupts said at least one DNA sequence identified in step c when it is bound to said recognition molecule, or
4) an expression vector, which comprises a nucleic acid sequence encoding a recognition molecule that specifically recognizes at least one DNA sequence identified in step c, and a molecule that selectively disrupts said at least one DNA sequence identified in step c when being bound to said recognition molecule, or
5) a recognition molecule that specifically recognizes at least one DNA sequence identified in step c and a molecule that selectively disrupts said at least one DNA sequence identified in step c when it is bound to said recognition molecule.

8. The method according to claim 7, wherein, for each time a sequence is identified in step c, the sequence is added to a list of potential target sequences, which is optionally ranked and re-ranked with reference to already included sequences and sequences that are continuously included.

9. The method according to claim 7 or 8, wherein step c includes ranking the sequences identified.

10. The method according to claim 9, which comprises 1) ranking sequence findings highest that have the biggest difference between the healthy and the cancerous/pathogen-related sequences and/or 2) ranking sequence finding(s) highest whose use is known to have the highest impact based on a predefined database of knowledge of the importance of genes.

11. The method according to any one of claims 7-10, wherein the DNA identified in step c are followed 1-20 nucleotides downstream or upstream by a proto-spacer adjacent motif (PAM) sequence.

12. A computer or computer system for designing and optionally preparing a therapeutic means as defined in any one of claims 7-11, comprising
i) a first computer memory or memory segment comprising DNA sequence data from healthy cells of an animal or from a fully sequenced healthy genome of said animal's species,
ii) a second computer memory or memory segment comprising DNA sequence data from malignant cells,
iii) executable code adapted to compare DNA sequence data from the second computer memory with the entire set of DNA sequence data in the first memory and identifying DNA sequences from the second computer memory, which 1) do not appear in DNA sequence data in the first computer memory and 2) are followed downstream or upstream by a proto-spacer adjacent motif (PAM) sequence,
iv) a third computer memory or memory segment which is adapted to comprise DNA sequence data, or pointers to DNA sequence data, identified by the computer defined in iii, and
v) executable code adapted to design expression vectors based on the 1) DNA sequence data stored or pointed to in the third computer memory or memory segment, and 2) sequence data for nucleic acids encoding effector molecules, and optionally
vi) a nucleic acid synthesizer adapted to synthesize DNA sequences designed by the executable code in v.

13. The computer or computer system according to claim 12, configured to use the provided design(s) and/or sequence(s) in the synthesis of CRISPR-Cas9 system(s), such as those involving Cas9, eSpCas9, SpCas9-HF, or Cpf1, alternatively homologous recombination, RNA interference (RNAi), zinc-finger nucleases (ZFNs), and transcription-activator like effector nucleases (TALENs) to make precise, targeted changes to the genome.

14. The computer or computer system according to claim 12 or 13, which carries out the method according to claim 6, steps b-d.

15. The computer or computer system according to any one of claims 12-14, wherein said DNA sequences identified in iii are followed 1-20 nucleotides downstream or upstream by a proto-spacer adjacent motif (PAM) sequence.
